# EUROPÄISCHE PATENTSCHRIFT

(11) **EP 1 305 378 B1**
(45) Veröffentlichungstag und Bekanntmachung des Hinweises auf die Patenterteilung: **16.11.2005**
(21) Anmeldenummer: 01956526.6
(22) Anmeldetag: 11.07.2001
(51) Int. Cl.: C09J 133/08, A61L 15/58

(54) **MEDIZINISCHER HAFTKLEBER MIT EINER ZWEIPHASIGEN KLEBERMATRIX AUS POLYACRYLATEN UND POLYAMINSALZEN**
MEDICAL CONTACT ADHESIVE COMPRISING A TWO PHASE ADHESIVE MATRIX OF POLYACRYLATES AND POLYAMINE SALTS
ADHESIF MEDICAL A MATRICE ADHESIVE A DEUX PHASES A BASE DE POLYACRYLATES ET DE SELS DE POLYAMINE

(30) Priorität: 24.07.2000 DE 10035891
(43) Veröffentlichungstag der Anmeldung: 02.05.2003
(73) Patentinhaber: LTS LOHMANN Therapie-Systeme AG, 56626 Andernach (DE)
(72) Erfinder: KOCH, Andreas, 56581 Melsbach (DE); BRACHT, Stefan, 56299 Ochtendung (DE); SCHMITZ, Christoph, 56598 Rheinbrohl (DE)
(74) Vertreter: Flaccus, Rolf-Dieter
(86) Internationale Anmeldenummer: PCT/EP2001/007982
(87) Internationale Veröffentlichungsnummer: WO 2002/008352

(56) Entgegenhaltungen:
- US-A- 5 977 242
- DATABASE WPI Section Ch, Week 199506 Derwent Publications Ltd., London, GB; Class A14, AN 1995-041540 XP002183812 & JP 06 322342 A (KONISHI CO LTD), 22. November 1994 (1994-11-22)

## Beschreibung

Die vorliegende Erfindung betrifft Haftkleber auf Polyacrylat-Basis für medizinische Zwecke, die bei der Herstellung von Heftpflastern, transdermalen therapeutischen Systemen (TTS) und anderen auf der Haut klebenden Vorrichtungen verwendet werden. Sie betrifft insbesondere wirkstoffhaltige Haftkleber für den Einsatz in transdermalen therapeutischen Systemen.
Des weiteren umfaßt die Erfindung auch die Verwendung solcher Haftkleber zur Herstellung von Vorrichtungen, die für therapeutische oder diagnostische Anwendungszwecke dauerhaft auf der Haut befestigt werden.

Haftkleber sind hochviskose, elastische Klebstoffe, die nach einem kurzen, leichten Anpreßdruck sofort und dauerhaft auf dem jeweiligen Substrat, beispielsweise auf der Haut, kleben bleiben. Aus diesem Grund werden sie auch oft als "druckempfindliche Haftkleber" ("pressure sensitive adhesives" = PSA) bezeichnet. Sie können sich aufgrund ihrer Viskoelastizität sehr gut auf die Haut verschiedener Körperzonen anpassen und sind deshalb für eine Vielfalt von medizinischen Anwendungszwecken geeignet. Außerdem lassen sie sich in den meisten Fällen zu einem späteren Zeitpunkt wieder vom Substrat ablösen, ohne daß dieses dadurch zerstört wird.
Bei transdermalen therapeutischen Systemen können Haftkleber sowohl die Funktion eines Wirkstoffreservoirs in Form einer Polymermatrix, in welche der Wirkstoff eingebracht ist, als auch die Funktion der haftklebenden Befestigung des Systems auf der Haut besitzen.

Die derzeit am häufigsten eingesetzten Haftkleber basieren auf synthetischen Kautschukpolymeren, Polyacrylaten oder Silikonen. Haftkleber auf der Basis von synthetischen Kautschukpolymeren erfordern in der Regel den Zusatz von sogenannten "Klebrigmachern", z. B. klebrigmachenden Harzen auf der Basis von Kolophoniumderivaten, damit eine ausreichende bioadhäsive Haftung auf der menschlichen Haut erreicht werden kann. Im Gegensatz dazu sind Haftkleber auf der Basis von Polyacrylatpolymeren oder Silikonen aufgrund ihrer chemischen Struktur und den daraus resultierenden physikochemischen Eigenschaften selbstklebend, ohne daß ein Zusatz von klebrigmachenden Stoffen nötig ist.

Bei Haftklebern auf der Basis von synthetischen Kautschukpolymeren und klebrigmachenden Harzen wird oft eine mangelnde Hautverträglichkeit beobachtet. Hingegen ist das Problem der Hautverträglichkeit bei Polyacrylat- oder Silikonhaftklebern weitestgehend gelöst.

Jedoch erfüllen auch die derzeit bekannten Polyacrylatpolymere und Silikone nicht alle Anforderungen, die an medizinische Hochleistungskleber, beispielsweise zur Verwendung in transdermalen therapeutischen Systemen, gestellt werden. Hervorzuheben ist dabei vor allem, daß die Hauthaftung des Haftklebers auch unter Extrembedingungen erhalten bleiben muß, z. B. auch bei extremen klimatischen Bedingungen oder bei erhöhter und anhaltender Hautfeuchtigkeit, wie sie z. B. durch starkes Schwitzen hervorgerufen wird. Eine vermehrte Feuchtigkeitsbildung der Haut kann auch durch den Okklusiveffekt hervorgerufen werden, der durch ein auf die Haut appliziertes TTS bewirkt wird.

Insbesondere bei Silikonklebern ist außerdem das Problem des "kalten Flusses" noch nicht befriedigend gelöst. Man versteht darunter - im Zusammenhang mit TTS oder Heftpflastern - ein seitliches Auseinanderfließen gestanzter Pflaster-Flächen an den Schnittkanten aufgrund unzureichender Kohäsion. Dies kann insbesondere beim Konfektionieren zu großen Problemen führen, beispielsweise durch irreversibles Verkleben der Haftmatrix mit dem Verpackungsmaterial. Eine weitere unerwünschte Auswirkung des "kalten Flusses" besteht darin, daß der Haftkleber bei der Applikation auf der Haut unerwünscht tief in die Poren der Haut eindringt, wodurch die spätere Wiederablösung des Pflasters bzw. des TTS erschwert wird oder mit Schmerzen verbunden ist.

Haftkleber auf der Basis von Polyacrylatpolymeren weisen häufig das Problem auf, daß sie den Zusatz von Klebkraftmodulatoren, z. B. von sogenannten Weichmachern, erfordern, damit auch unter den oben beschriebenen extremen Bedingungen eine ausreichende Klebwirkung gewährleistet werden kann. Dazu müssen die Polyacrylat-Haftkleber sehr weich eingestellt werden, was häufig zur Folge hat, daß ein Zurückbleiben von Kleberresten auf der Haut nach dem Wiederablösen des Pflasters nicht vermieden werden kann.

Speziell bei Haftklebern, die saure Polyacrylate enthalten, ergeben sich weitere Probleme dadurch, daß sie eine geringe Toleranz gegen Feuchtigkeit aufweisen, was Nachteile bezüglich der Hauthaftung nach sich zieht, und daß sie eine chemische Affinität zu basischen Wirkstoffen aufweisen. Letzteres ist nachteilig, weil infolge einer irreversiblen Säure-Base-Reaktion zwischen den sauren Polyacrylaten und den basischen Wirkstoffen die Freisetzung und damit die Bioverfügbarkeit des betreffenden Arzneistoffes erniedrigt wird.

Deshalb sollte ein Haftkleber, um für den Einsatz in transdermalen therapeutischen Systemen geeignet zu sein, idealerweise in physiko-chemischer Hinsicht kompatibel mit den verschiedensten Arzneiwirkstoffen sein und keine ungünstigen Wechselwirkungen mit diesen eingehen.
Ferner sollte ein Haftkleber folgende vorteilhafte Klebeigenschaften aufweisen: er sollte über eine gewisse Mindestklebrigkeit verfügen, ohne daß hierfür ein Weichmacherzusatz erforderlich ist. Diese Mindestklebrigkeit sollte erst unter Einwirkung der Hautfeuchtigkeit in eine aggressive Klebrigkeit übergehen, aber dennoch wasserresistent sein. Des weiteren ist zu fordern, daß sich der Haftkleber nach Beendigung der Applikation rückstandsfrei von der Haut entfernen läßt.

In DE 44 29 791 A1 wird eine Haftkleberzusammensetzung beschrieben, die zwei Polymerkomponenten enthält, nämlich ein selbstklebendes, carboxylgruppenhaltiges Polyacrylatpolymer und ein basische Aminogruppen enthaltendes Polymer. Auf diese Weise soll durch Wechselwirkungen zwischen den sauren und basischen Gruppen eine Vernetzung herbeigeführt werden. Unter dem Einfluß der Hautfeuchtigkeit werden diese Wechselwirkungen geschwächt, was eine erwünschte Zunahme der Klebrigkeit während der Applikationsdauer zur Folge hat. Allerdings ist die Anfangsklebrigkeit eines solchen Haftklebers sehr gering, weshalb zusätzlich Weichmacher beigefügt werden müssen, um die erforderliche Mindest- oder Grundklebrigkeit zu erreichen. Die zugesetzten Weichmacher können allerdings zu den oben beschriebenen Nachteilen führen.
Da die beschriebene Wechselwirkung zwischen den sauren und basischen Gruppen unter dem Einfluß von Feuchtigkeit reversibel ist, kann es bei Verwendung in einem TTS zu unerwünschten Wechselwirkungen mit den gegebenenfalls anwesenden sauren oder basischen Wirkstoffen kommen, mit nachteiligen Auswirkungen auf deren Freisetzung an die Haut und somit auf die Bioverfügbarkeit.

Des weiteren ist vorgeschlagen worden, saure Polyacrylathaftkleber zur Verbesserung der Klebeigenschaften mittels Alkalimetallverbindungen zu neutralisieren, jedoch nur in Verbindung mit sauren pharmazeutischen Wirkstoffen. Durch die Neutralisation der sauren Polyacrylathaftkleber soll eine Steigerung der Kohärenz, eine verbesserte Hauthaftung, eine erhöhte Beladbarkeit mit weichmachenden Hilfsstoffen sowie eine erhöhte Beständigkeit gegen Feuchtigkeit bezweckt werden. Derartige Haftkleber haben allerdings den Nachteil, daß sie als Wirkstoffreservoir für beispielsweise hydrolyseanfällige oder chemisch instabile pharmazeutische Wirkstoffe wenig geeignet sind, da das leicht alkalische Milieu dieses Haftklebersystems die Gefahr einer beschleunigten Zersetzung bzw. eines verstärkt ablaufenden Abbaues der Wirkstoffe mit sich bringt.
Außerdem erwiesen sich neutralisierte Polyacrylatpolymere wider Erwarten doch zum Teil als feuchtigkeitsempfindlich und, bedingt durch ihre hohe Kohäsion und der damit verbundenen geringen Fließfähigkeit, in ihrer Klebkraft nachlassend.
Aus diesem Grund sind bei Haftklebersystemen auf der Basis neutralisierter Polyacrylate stark wasserbindende Zusätze zur Erhöhung der Feuchtigkeitstoleranz erforderlich. Darüber hinaus ist für die Erzielung einer optimalen Hauthaftung der Einsatz von Weichmachern notwendig, oder das Hinzufügen einer zusätzlichen Haftkleberschicht, die die Verklebung des Systems mit der Haut verbessert.

Aufgabe der vorliegenden Erfindung war es deshalb, medizinische Haftkleber bereitzustellen, welche für die genannten Einsatzgebiete, insbesondere für die Verwendung in transdermalen therapeutische Systeme, geeignet sind, und
- die während der Herstellung und Lagerung nicht zu "kaltem Fluß" neigen,
- die über eine ausreichend druckempfindliche Hauthaftung und gute Anfangsklebrigkeit verfügen,
- deren Hauthaftung während der gesamten Applikationsdauer unter dem Einfluß von Hautfeuchtigkeit erhalten bleibt oder sich sogar noch steigert,
- die aufgrund ihrer Wasser- bzw. Feuchtigkeitsresistenz nach Ende der Therapie rückstandsfrei von der Haut abgelöst werden können, d. h. ohne daß Kleberreste zurückbleiben, und
- die zu sauren oder basischen Arzneiwirkstoffen chemisch inert bzw. mit diesen kompatibel sind, und insbesondere keine Wechselwirkungen, beispielsweise durch Salzbildung oder Wasserstoffbrückenbindungen, eingehen.

Überraschenderweise wird diese Aufgabe durch einen Haftkleber gemäß Anspruch 1 gelöst, sowie durch die in den Unteransprüchen beschriebenen Ausführungsformen. Dies ist vor allem deshalb unerwartet, da neutrale Polyaminsalze (Komponente B) ein zu großes Wasseraufnahmevermögen und Quellvermögen aufweisen und deshalb als solche nicht zur Verwendung als Haftkleber geeignet erscheinen, weil dadurch die Klebkraft verschlechtert und die Freisetzung insbesondere von hydrophilen Wirkstoffen aus einer solchen Klebmatrix beeinträchtigt wird. Eine rückstandsfreie Ablösung derartiger Kleber von der Haut ist nicht möglich.

Der erfindungsgemäße Haftkleber weist eine Klebmatrix auf, die eine Kombination aus zwei Polymerkomponenten aufweist, die als polymerkomponenten A und B bezeichnet werden.
Bei Polymerkomponente A handelt es sich um einen carboxylgruppenhaltigen Polyacrylathaftkleber, dessen Carboxylgruppen neutralisiert sind. Dabei wird ein Polyacrylathaftkleber bevorzugt, der mindestens 3 Mol-% einpolymerisierte Acryl- oder Methacrylsäure enthält. Die Neutralisation der Carboxylgruppen wird vorzugsweise durch Alkalilaugen bewirkt.

Polymerkomponente B ist ein neutrales Polyaminsalz, welches durch Umsetzung eines aminogruppenhaltigen Polyacrylat-Copolymers mit einer Säure erhalten wurde. Bevorzugt ist das aminogruppenhaltige Polyacrylat ein Poly(meth)acrylatcopolymer aus neutralen Methacrylsäureestern und Dimethylaminoethylmethacrylat (z. B. Eudragit E 100, Fa. Röhm).

Als Säuren, mit welchen das aminogruppenhaltige Polyacrylat bzw. das aminogruppenhaltige Poly(meth)acrylatcopolymer umgesetzt wird, werden bevorzugt Fettsäuren und/oder Dicarbonsäuren verwendet, wobei auch ein Gemisch von Säuren eingesetzt werden kann. Als Fettsäuren werden bevorzugt ungesättigte Fettsäuren verwendet, besonders bevorzugt mit einer Kettenlänge von 6 bis 20 C-Atomen. Als Dicarbonsäuren werden vorzugsweise gesättigte Dicarbonsäuren mit 4 bis 10 C-Atomen verwendet.

Die aus den beiden Komponenten gebildete Klebmatrix stellt ein zweiphasiges System dar, bei welchem die Polymerkomponente B in der Polymerkomponente A dispergiert ist, ähnlich wie bei einer Wasser-in-Öl-Emulsion. Dies beruht darauf, daß die Polymerkomponente A wasserunlöslich ist, während die Polymerkomponente B (Polyaminsalz) wasserlöslich ist.
Das quaternäre Polymethacrylat (Polyaminsalz) ist hydrophil und ionisch, und damit sehr gut wasserlöslich.

Die Polymerkomponente B ist in der Komponente A dispergiert, wobei sich Bereiche ausbilden, die als "Mikro-Reservoire" betrachtet werden können. Wird der erfindungsgemäße Haftkleber, wie vorgesehen, in beiden Komponenten mit Wirkstoffen beladen, so verteilt sich der Wirkstoff zwischen der Komponenten A und den Mikro-Reservoirs B, die in der polymerkomponenten A dispergiert sind. Die Verteilung des Wirkstoffs zwischen den beiden Polymerkomponenten gestaltet sich entsprechend dem Verteilungskoeffizienten des betreffenden Arzneimittelwirkstoffes, beispielsweise bezogen auf das System Octanol/Wasser.

Die beiden Polymerkomponenten können in unterschiedlichen Gewichtsanteilen vermischt bzw. dispergiert werden, um das erwähnte zweiphasige System zu erhalten. Bevorzugt wird dabei ein Verhältnis der Komponente A zur Komponente B von 0,5 : 1 bis 50 : 1, bezogen auf Gewichtsanteile. Besonders bevorzugt ist ein Verhältnis von 1 : 1 bis 10 : 1.

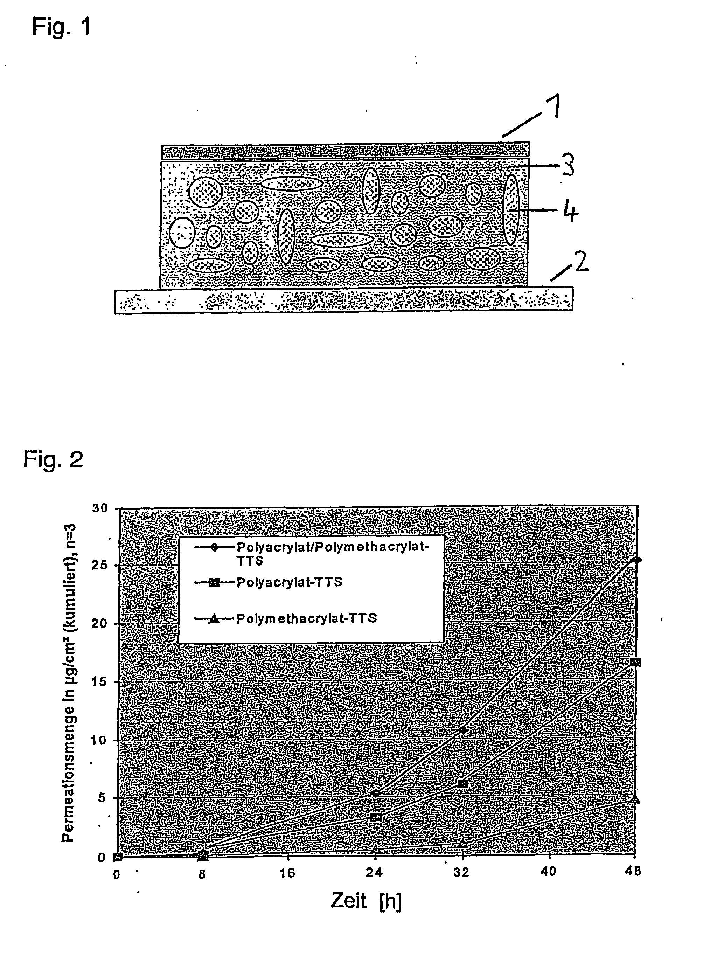

Ein weiterer Vorteil der erfindungsgemäßen medizinischen Haftkleber ist darin zu sehen, daß auf die Verwendung von Weichmachern, die sonst aus technologisch bedingten Gründen oft üblich ist, verzichtet werden kann.
Das erfindungsgemäß vorgeschlagene Klebersystem verfügt über hervorragende Hauthaftungseigenschaften auch unter schwierigsten Bedingungen und weist keinen "kalten Fluß" auf. Ferner kann ein Pflaster oder TTS, welches mit dem erfindungsgemäßen Haftkleber ausgestattet ist, bei Beendigung der Applikationsdauer völlig rückstandsfrei von der Haut entfernt werden. Gegenüber Haftklebern, die ausschließlich auf der Basis von Polyacrylaten oder von Polyaminsalzen hergestellt sind, besitzt ein erfindungsgemäßer Haftkleber auch bessere Freisetzungseigenschaften für die darin gelösten oder dispergierten Wirkstoffe (siehe Fig. 2).
Ein weiterer Vorteil der erfindungsgemäßen Haftkleber besteht darin, daß für ihre Herstellung Polyacrylate als Ausgangsstoffe verwendet werden können, die in der pharmazeutischen Technologie bereits gut bekannt und kommerziell erhältlich sind, und die von der FDA zugelassen sind (siehe Beispiel 1).

Der erfindungsgemäße medizinische Haftkleber kann, wie erwähnt, mit pharmazeutischen Wirkstoffen beladen werden, so daß zumindest eine der beiden Polymerkomponenten einen Wirkstoffgehalt aufweist. Die erzielbare Beladungskapazität ist dabei vergleichbar mit derjenigen von Polyacrylatpolymeren nach dem Stand der Technik. Jedoch kann bei den erfindungsgemäßen Haftklebern auf die sonst benötigten Lösungsvermittler für den Arzneiwirkstoff, deren Funktion auch von Weichmachern übernommen werden kann, verzichtet werden.
Vorzugsweise enthalten die beiden Polymerkomponenten A und B denselben pharmazeutischen Wirkstoff. In besonderen Fällen kann es auch vorteilhaft sein, wenn die Komponenten A und B jeweils mit unterschiedlichen Wirkstoffen beladen werden.

Eine zusätzliche Quervernetzung des Polyacrylatpolymer-Gerüsts zur Vermeidung des "kalten Flusses" ist bei den erfindungsgemäßen Haftklebern nicht nötig. Falls gewünscht, kann eine solche Vernetzung durch Zusatz von Metallchelaten, z. B. von Aluminiumacetylacetonat, bewirkt werden.

Um die Hautpenetration der in die Haftklebematrix eines TTS eingebrachten Wirkstoffe zu verbessern, können dem erfindungsgemäßen medizinischen Haftkleber, welcher die genannte Haftklebematrix des TTS bildet, sogenannte Penetrationsförderer ("Enhancer") zugesetzt werden, wie z. B. gesättigte oder ungesättigte Fettsäuren, geradkettige oder verzweigte Fettalkohole bzw. deren Ester, mehrwertige aliphatische Alkohole oder Polyethylenglykole, Sorbitanfettsäureester und deren durch Ethoxylierung erhältlichen Derivate, oder Fettalkoholethoxylate.
Der Anteil dieser Penetrationsförderer beträgt vorzugsweise 5-20 Gew.-%.

Alle Angaben, welche Gewichtsprozent-Anteile angeben, beziehen sich im Zweifelsfalle auf das Gewicht des Haftklebers nach Entfernen der bei der Herstellung zugesetzten Lösungsmittel.

In einer besonders bevorzugten Ausführungsform enthält der erfindungsgemäße medizinische Haftkleber 60-95 Gew.-% eines zweiphasigen, neutralisierten Haftklebers und 1-20 Gew.-% Wirkstoff, der in dem zweiphasigen Haftkleber gelöst oder dispergiert vorliegt. Dabei ist die Polymerkomponente A des zweiphasigen Haftklebers ein neutralisiertes Polyacrylatcopolymer mit mindestens 3 Molprozenten einpolymerisierter Acryl- oder Methacrylsäure, und die Polymerkomponente B des zweiphasigen Haftklebers ist ein neutrales Polyaminsalz, welches durch Umsetzung eines Poly(meth)acrylatcopolymers aus neutralen Methacrylsäureestern und Dimethylaminoethylmethacrylat erhalten wurde.
Ein derartiger wirkstoffhaltiger medizinischer Haftkleber ist vorzugsweise als Wirkstoffmatrix eines transdermalen therapeutischen Systems geeignet.

Als Wirkstoffe, mit denen die erfindungsgemäßen medizinischen Haftkleber beladen werden können, und die nachfolgend während der Applikation an die Haut eines Patienten abgegeben werden können, werden bevorzugt saure und/oder basische pharmazeutische Wirkstoffe verwendet, oder deren pharmazeutisch akzeptablen Salze.

Unter basischen pharmazeutischen Wirkstoffen sind solche zu verstehen, die in ihrer Molekülstruktur mindestens eine Gruppe aufweisen, die chemisch als Lewis-Base reagieren kann. Vorzugsweise weisen solche Gruppen eine aliphatische oder aromatische primäre, sekundäre oder tertiäre Aminfunktion auf. Beispiele sind natürlich vorkommende Alkaloide wie Atropin, Scopolamin, Physostigmin, Galanthamin, Pilocarpin, Morphin oder Ergotamin oder synthetisch hergestellte Wirkstoffe wie Apomorphin, Moxonidin, Desoxypeganin, Buprenorphin, Salbutamol oder Clonidin.

Unter sauren pharmazeutischen Wirkstoffen sind solche zu verstehen, die in ihrer Molekülstruktur mindestens eine Gruppe aufweisen, die chemisch als Lewis-Säure reagieren kann. Vorzugsweise weisen solche Gruppen eine azide Carbonylfunktion auf. Beispiele sind die Carbonsäuren Acetylsalicylsäure, Dehydrocholsäure, Ketoprofen, Lovastatin und Flurbiprofen.
Abgesehen von sauren oder basischen Wirkstoffen können aber auch andere Wirkstoffe, die für die transdermale Verabreichung geeignet sind, in die erfindungsgemäßen medizinischen Haftkleber eingearbeitet werden, um Wirkstoffmatrices für transdermale therapeutische Systeme herzustellen.

Die erfindungsgemäßen medizinischen Haftkleber sind überall dort einsetzbar, wo ein gutes und zuverlässiges Kleben übe einen längeren Zeitraum auf der Haut gefordert ist. Sie eignen sich deshalb besonders zur Herstellung von Wundschnellverbänden, Fixierpflastern, Heftpflastern, Wundpflastern, oder selbstklebenden Elektroden.
Besonders vorteilhaft können diese Haftkleber zur Herstellung von transdermalen therapeutischen Systemen in Pflasterform für die transdermale Verabreichung von pharmazeutischen Wirkstoffen verwendet werden. Der Aufbau eines solchen TTS ist in Fig. 1 schematisch dargestellt.

Die erfindungsgemäßen medizinischen Haftkleber und ihre vorteilhaften Eigenschaften werden nachfolgend anhand von Beispielen und Zeichnungen erläutert.

### Beispiel 1

Herstellung eines selbstklebenden, wirkstoffhaltigen Haftklebefilms:

### Komponente A:

Ein saurer, freie Carboxylgruppen enthaltender Polyacrylatkleber (z. B. Durotak® 387-2051; in Ethylacetat/n-Heptan; 52 Gew.-%; Fa. National Starch) wird entsprechend seiner Säure-Zahl von 37-44 mg KOH pro Gramm Trockengewicht unter Rühreintrag mit einer 10%igen (Gew.-%) ethanolischen oder methanolischen Lösung von Natrium- oder Kaliumhydroxid neutralisiert. Die dabei auftretende Viskositätserhöhung kann durch Lösemittelzugabe (Ethylacetat/n-Heptan/Methanol) ausgeglichen werden. Optional kann der so erhaltenen, neutralisierten Acrylatklebermasse Aluminiumacetylacetonat (4 Gew.-% in Ethylacetat) als Quervernetzer in einem Konzentrationsbereich von 0,005 bis 0,5 Gew.-%, bezogen auf Aluminium, zugegeben werden.

### Komponente B:

Eudragit® E 100 (Fa. Röhm; 15,9 Gewichtsteile, entsprechend 57 Gew.-%) wird in Ethanol gelöst und unter Rühreintrag mit Fettsäuren zu einem Polyaminsalz umgesetzt. Als Fettsäuren können z. B. Laurinsäure (9,4 Teile, entsprechend 33,5 Gew.-%) und Adipinsäure (1,86 Teile, entsprechend 6,6 Gew.-%) eingesetzt werden.

Anschließend werden die so erhaltenen Komponenten A und B im Verhältnis 1:1 (bezogen auf Feststoff-Gewicht) gemischt und durch Rühren homogenisiert. In 13,03 g der 29,5%igen vorgemischten Polyacrylat/Polyaminsalz-Kleberlösung wird unter Rühren portionsweise 0,665 g Morphiniumbenzoat (13,1 Gew.-%) als Wirkstoff und 0,5 g Ölsäure als Enhancer eingetragen. Zwecks Erniedrigung der Viskosität des Masseansatzes kann Methanol zugesetzt werden. Der Masseansatz wird insgesamt 30 min bei einer Rührgeschwindigkeit von 350 U/min homogenisiert. Anschließend erfolgt eine viertelstündige Entgasung bei 45 °C im Ultraschallbad, um überschüssige Luft aus der Masse zu entfernen.
Die wirkstoffhaltige Kleberlösung wird dann mit Hilfe einer Ausziehrakel in einer Naßschichtdicke von 300 µm auf einer silikonisierten Polyethylenterephthalat-Folie ausgestrichen. Danach werden die Lösemittel durch halbstündiges Trocknen bei 50 °C in einem Trockenschrank mit Abluftführung entfernt. Der lösemittelfreie, wirkstoffhaltige Klebefilm wird abschließend mit einer 15 µm dicken Polyesterfolie durch Aufkaschieren abgedeckt. Der Kleberanteil im Laminat nach Fertigungsende beträgt 76,9 Gew.-%. Das Laminat weist die Grundstruktur einer zweiphasigen TTS-Haftklebermatrix auf, wie sie in Fig. 1 schematisch dargestellt ist.

Fig. 1 zeigt im Querschnitt den schematischen Aufbau eines TTS mit einer erfindungsgemäßen zweiphasigen Haftklebematrix als Wirkstoffreservoir.
Die einzelnen Bestandteile des TTS sind wie folgt bezeichnet:
(1) wirkstoffundurchlässige Rückschicht ("backing layer");
(2) ablösbare Schutzfolie ("protective layer" oder "release liner");
(3) selbstklebende Polyacrylatmatrix (neutralisierter carboxylgruppenhaltiger Polyacrylathaftkleber; Komponente A), mit Wirkstoff;
(4) darin dispergiertes Poly(meth)acrylat (neutrales Polyaminsalz; Komponente B), ebenfalls mit Wirkstoff. Das dispergierte Poly(meth)acrylat bildet "Mikro-Reservoirs".

Der gezeigte Aufbau eines TTS soll lediglich als Beispiel dienen. Die Erfindung erstreckt sich auch auf Ausführungsvarianten von TTS, die von dem gezeigten Schema abweichen.

Als Materialien für die Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium.

Für die ablösbare Schutzschicht können grundsätzlich dieselben Materialien verwendet werden wie für die Rückschicht, vorausgesetzt, daß sie durch eine geeignete Oberflächenbehandlung, wie z. B. Silikonisierung, ablösbar ausgerüstet ist. Es können aber auch andere ablösbaren Schutzschichten wie z. B. mit Polytetrafluorethylen behandeltes Papier oder Cellophan® (Cellulosehydrat) verwendet werden.

### Beispiel 2

Entsprechend der in Beispiel 1 beschriebenen Vorgehensweise wurde ein erfindungsgemäßer Haftkleber mit einem Polymergehalt von 86,9 Gew.-% und einem Wirkstoff-Gehalt von 13,1 Gew.-% hergestellt. Der Haftkleber besteht, wie unter Beispiel 1 beschrieben, aus Durotak® 387-2051 (neutralisiert) und neutralem Polyaminsalz (Eudragit® E 100, versalzt mit Laurin- und Adipinsäure). Als Wirkstoff wurde Morphiniumbenzoat eingesetzt.
Mit dieser wirkstoffhaltigen Haftklebermatrix (Kurzbezeichnung: "Polyacrylat/Polymethacrylat-TTS") wurde am in-vitro-Hautmodell (humane Vollhaut, weibliche Brust, Alter: 23 Jahre) die Wirkstoff-Permeation untersucht. Als Akzeptormedium wurde 0,9 %ige NaCl-Lösung (mit 0,1 Gew.-% NaN₃) verwendet. Die Temperatur während der Dauer des Freisetzungsexperiments betrug 37 °C.

Die dabei ermittelten Permeationswerte sind in Fig. 2 graphisch dargestellt.

Als Vergleichsbeispiele wurden die folgenden Haftklebermatrices hergestellt, welche ebenfalls einen Wirkstoffgehalt (Morphiniumbenzoat) von 13,1 Gew.-% aufwiesen:
a) Haftklebermatrix mit 61,9 Gew.-% saurem Durotak® 387-2051-Haftkleber (nicht neutralisiert) und 25 Gew.-% Ölsäure;
   Kurzbezeichnung: "Polyacrylat-TTS"
b) Haftklebermatrix mit 86,9 Gew.-% neutralem Polyaminsalz-Haftkleber (Eudragit® E 100, versalzt mit Laurin- und Adipinsäure);
   Kurzbezeichnung: "Polymethacrylat-TTS".

Mit den als Vergleichsbeispielen herangezogenen Haftklebermatrices "Polyacrylat-TTS" und "Polymethacrylat-TTS" wurde, wie oben beschrieben, die Wirkstoffpermeation an menschlicher Vollhaut untersucht. Die Ergebnisse dieser Permeationsexperimente sind ebenfalls in Fig. 2 dargestellt.

Es zeigte sich, daß mit dem erfindungsgemäßen Haftkleber ("Polyacrylat/Polymethacrylat-TTS") eine gegenüber den Vergleichsbeispielen deutlich verbesserte Hautpermeation erzielt wurde.
Da die erfindungsgemäßen Haftkleber sich nicht nur durch eine gesteigerte Wirkstofffreisetzung, sondern auch durch verbesserte Klebeigenschaften auszeichnen, erfüllen sie die Anforderungen, die an einen medizinischen Haftkleber zu stellen sind, in vorzüglicher Weise.

## Patentansprüche

1. Medizinischer Haftkleber auf der Basis von Polyacrylaten und neutralen Polyaminsalzen, **dadurch gekennzeichnet, daß** er eine Haftmatrix aus zwei Polymerkomponenten A und B aufweist, wobei
- Polymerkomponente A ein carboxylgruppenhaltiger Polyacrylathaftkleber ist, dessen Carboxylgruppen neutralisiert sind;
- Polymerkomponente B ein neutrales Polyaminsalz ist, welches durch Umsetzung eines aminogruppenhaltigen Polyacrylat-Copolymers mit einer Säure erhalten wurde, und wobei
- die Polymerkomponente B in der Polymerkomponente A dispergiert ist und die beiden Komponenten ein zweiphasiges System bilden.

2. Haftkleber nach Anspruch 1, **dadurch gekennzeichnet, daß** als Säure für die Umsetzung des aminogruppenhaltigen Polyacrylats eine Säure oder ein Gemisch von Säuren verwendet wird, die ausgewählt ist/sind aus der Gruppe der ungesättigten Fettsäuren und der Dicarbonsäuren.

3. Haftkleber nach Anspruch 1 oder 2, **dadurch gekennzeichnet, daß** das aminogruppenhaltige Polyacrylat ein Poly-(meth)acrylatcopolymer aus neutralen Methacrylsäureestern und Dimethylaminoethylmethacrylat ist.

4. Haftkleber nach einem der Ansprüche 1 bis 3, **dadurch gekennzeichnet, daß** der carboxylgruppenhaltige Polyacrylathaftkleber mindestens 3 Mol-% einpolymerisierte Acryl- oder Methacrylsäure enthält.

5. Haftkleber nach einem oder mehreren der Ansprüche 1 bis 4, **dadurch gekennzeichnet, daß** die Carboxylgruppen des Polyacrylathaftklebers mit Alkalilaugen neutralisiert sind, vorzugsweise mit NaOH oder KOH.

6. Haftkleber nach einem oder mehreren der Ansprüche 1 bis 5, **dadurch gekennzeichnet, daß** Komponente A und B im Verhältnis 0,5 : 1 bis 50 : 1 dispergiert sind, bevorzugt im Verhältnis 1 : 1 bis 10 : 1, jeweils bezogen auf das Gewicht.

7. Haftkleber nach einem oder mehreren der Ansprüche 1 bis 6, **dadurch gekennzeichnet, daß** der neutralisierte carboxylgruppenhaltige Polyacrylatkleber durch Zusatz von Metallchelaten, vorzugsweise von Aluminiumacetylacetonat, vernetzt ist.

8. Haftkleber nach einem oder mehreren der Ansprüche 1 bis 7, **dadurch gekennzeichnet, daß** zumindest eine der beiden Polymerkomponenten, vorzugsweise aber beide Polymerkomponenten, wirkstoffhaltig ist bzw. sind, wobei der/die Wirkstoff(e) in den Polymerkomponenten gelöst oder suspendiert vorliegen.

9. Haftkleber nach Anspruch 8, **dadurch gekennzeichnet, daß** er
- 60-95 Gew.-% eines zweiphasigen, neutralisierten Haftklebers - wobei die Polymerkomponente A ein neutralisiertes Polyacrylatcopolymer mit mindestens 3 Molprozenten einpolymerisierter Acryl- oder Methacrylsäure ist, und wobei die Polymerkomponente B ein neutrales Polyaminsalz ist, welches durch Umsetzung eines Poly(meth)acrylatcopolymers aus neutralen Methacrylsäureestern und Dimethylaminoethylmethacrylat erhalten wurde - ; und
- 1-20 Gew.-% Wirkstoff, der in dem zweiphasigen Haftkleber gelöst oder dispergiert vorliegt,
enthält.

10. Haftkleber nach Anspruch 8 oder 9, **dadurch gekennzeichnet, daß** er einen oder mehrere penetrationsfördernde Zusatzstoffe enthält, die in den Polymerkomponenten gelöst oder suspendiert sind, wobei der Anteil dieser Penetrationsförderer vorzugsweise 5-20 Gew.-% beträgt.

11. Transdermales therapeutisches System in Pflasterform, mit einer wirkstoffundurchlässigen Rückschicht, einem wirkstoffhaltigen Reservoir auf der Basis einer Polymermatrix und einer ablösbaren Schutzfolie, **dadurch gekennzeichnet, daß** das wirkstoffhaltige Reservoir einen Haftkleber nach einem der Ansprüche 1 bis 10 enthält.

12. Verwendung eines Haftklebers nach einem oder mehreren der Ansprüche 1 bis 7 zur Herstellung von Wundschnellverbänden, Fixierpflastern, Heftpflastern, Wundpflastern oder selbstklebenden Elektroden.

13. 'Verwendung eines Haftklebers nach einem oder mehreren der Ansprüche 1 bis 10 zur Herstellung von wirkstoffhaltigen Pflastern für die transdermale Verabreichung von pharmazeutischen Wirkstoffen.

14. Verwendung nach Anspruch 13, **dadurch gekennzeichnet, daß** die wirkstoffhaltigen Pflaster saure oder basische pharmazeutische Wirkstoffe oder deren pharmazeutisch akzeptablen Salze enthalten.

## Claims

1. Medical pressure sensitive adhesive based on polyacrylates and neutral polyamine salts, **characterized in that** it has a pressure-sensitive adhesive matrix of two polymer components A and B, with
- polymer component A being a polyacrylate pressure-sensitive adhesive containing carboxyl groups, the carboxyl groups of which are neutralised;
- polymer component B being a neutral polyamine salt obtained by reacting a polyacrylate copolymer containing amino groups with an acid, and with
- the polymer component B being dispersed in the polymer component A, and the two components forming a two-phase system.

2. Pressure sensitive adhesive according to Claim 1, **characterized in that** as the acid for reacting the amino groups-containing polyacrylate, an acid or a mixture of acids is used which is/are selected from the group of the unsaturated fatty acids and the dicarboxylic acids.

3. Pressure sensitive adhesive according to Claim 1 or 2, **characterized in that** the amino groups-containing polyacrylate is a poly(meth)acrylate copolymer of neutral methacrylic acid esters and dimethylaminoethyl methacrylate.

4. Pressure sensitive adhesive according to Claims 1 to 3, **characterized in that** the carboxyl groups-containing polyacrylate adhesive contains at least 3 mole percent of acrylic or methacrylic acid polymerised therein.

5. Pressure sensitive adhesive according to one or more of Claims 1 to 4, **characterized in that** the carboxyl groups of the polyacrylate pressure sensitive adhesive are neutralised with alkali lyes, preferably with NaOH or KOH.

6. Pressure sensitive adhesive according to one or more of Claims 1 to 5, **characterized in that** the components A and B are dispersed at a ratio of 0.5:1 to 50:1, preferably at a ratio of 1:1 to 10:1, in each case relative to the weight.

7. Pressure sensitive adhesive according to one or more of Claims 1 to 6, **characterized in that** the neutralised, carboxyl groups-containing polyacrylate adhesive is crosslinked by addition of metal chelates, preferably of aluminium acetyl acetonate.

8. Pressure sensitive adhesive according to one or more of Claims 1 to 7, **characterized in that** at least one of the two polymer components, preferably, however, both polymer components, contain(s) active substance, with the active substance(s) being present in the polymer components in dissolved or suspended form.

9. Pressure sensitive adhesive according to Claim 8, **characterized in that** it contains
- 60-95%-wt of a two-phase, neutralised pressure sensitive adhesive - with the polymer component A being a neutralised polyacrylate copolymer with at least 3 mole percent of acrylic or methacrylic acid polymerised therein, and with the polymer component B being a neutral polyamine salt obtained by converting a poly(meth)acrylate copolymer of neutral methacrylic acid esters and dimethylaminoethyl methacrylate-; and
- 1-20%-wt of active substance, present in the two-phase pressure-sensitive adhesive in dissolved or dispersed form.

10. Pressure sensitive adhesive according to Claim 8 or 9, **characterized in that** it contains one or more penetration-enhancing additives which are dissolved or suspended in the polymer components, with the portion of said penetration enhancers preferably being 5-20%-wt.

11. Transdermal therapeutic system in the form of a plaster having an active substance-impermeable backing layer, an active substance-containing reservoir based on a polymer matrix and a detachable protective layer, **characterized in that** the active substance-containing reservoir contains a pressure sensitive adhesive according to any one of Claims 1 to 10.

12. Use of a pressure sensitive adhesive according to one or more of Claims 1 to 7 for the manufacture of adhesive dressings, fixing plasters, adhesive plasters, wound plasters or self-adhesive electrodes.

13. Use of a pressure sensitive adhesive according to one or more of Claims 1 to 10 for the production of active substance-containing plasters for the transdermal administration of pharmaceutical active substances.

14. Use according to Claim 13, **characterized in that** the active substance-containing plasters contain acid or basic pharmaceutically active substances or the pharmaceutically acceptable salts thereof.

## Revendications

1. Auto-adhésif médical à base de polyacrylates et de sels de polyamine neutres, **caractérisé en ce qu'**il présente une matrice adhésive constituée par deux composants polymères A et B, dans lequel
- le composant polymère A est un auto-adhésif de polyacrylate contenant des groupes carboxyle, dont les groupes carboxyle sont neutralisés ;
- le composant polymère B est un sel de polyamine neutre, qui a été obtenu par transformation d'un copolymère de polyacrylate contenant des groupes amino avec un acide et où
- le composant polymère B est dispersé dans le composant polymère A et les deux composants forment un système à deux phases.

2. Auto-adhésif selon la revendication 1, **caractérisé en ce qu'**on utilise comme acide pour la transformation du polyacrylate contenant des groupes amino un acide ou un mélange d'acides qui est/sont choisi(s) dans le groupe des acides gras insaturés et des acides dicarboxyliques.

3. Auto-adhésif selon la revendication 1 ou 2, **caractérisé en ce que** le polyacrylate contenant des groupes amino est un copolymère de poly(méth)acrylate constitué par des esters neutres de l'acide méthacrylique et du méthacrylate de diméthylaminoéthyle.

4. Auto-adhésif selon l'une quelconque des revendications 1 à 3, **caractérisé en ce que** l'auto-adhésif à base de polyacrylate contenant des groupes carboxyle contient au moins 3% en mole d'acide acrylique ou méthacrylique sous forme copolymérisée.

5. Auto-adhésif selon l'une ou plusieurs des revendications 1 à 4, **caractérisé en ce que** les groupes carboxyle de l'auto-adhésif à base de polyacrylate sont neutralisés avec des lessives alcalines, de préférence avec du NaOH ou du KOH.

6. Auto-adhésif selon l'une ou plusieurs des revendications 1 à 5, **caractérisé en ce que** les composants A et B sont dispersés dans un rapport de 0,5 : 1 à 50 : 1, de préférence dans un rapport de 1 : 1 à 10 : 1, à chaque fois par rapport au poids.

7. Auto-adhésif selon l'une ou plusieurs des revendications 1 à 6, **caractérisé en ce que** l'adhésif à base de polyacrylate, contenant des groupes carboxyle neutralisés est réticulé par addition de chélates de métaux, de préférence d'acétylacétonate d'aluminium.

8. Auto-adhésif selon l'une ou plusieurs des revendications 1 à 7, **caractérisé en ce qu'**au moins un des deux composants polymères, de préférence toutefois les deux composants polymères, contien(nen)t une ou plusieurs substance(s) active(s), la ou les substances actives se trouvant sous forme dissoute ou en suspension dans les composants polymères.

9. Auto-adhésif selon la revendication 8, **caractérisé en ce qu'**il contient
- 60 à 95% en poids d'un auto-adhésif à deux phases, neutralisé - le composant polymère A étant un copolymère de polyacrylate neutralisé comprenant au moins 3% en mole d'acide acrylique ou méthacrylique sous forme copolymérisée et le composant polymère B étant un sel de polyamine neutre, qui a été obtenu par transformation d'un copolymère de poly(méth)acrylate constitué par des esters neutres de l'acide méthacrylique et du méthacrylate de diméthylaminoéthyle ; et
- 1 à 20% en poids de substance active, qui se trouve sous forme dissoute ou dispersée dans l'auto-adhésif à deux phases.

10. Auto-adhésif selon la revendication 8 ou 9, **caractérisé en ce qu'**il contient un ou plusieurs additifs favorisant la pénétration, qui sont dissous ou en suspension dans les composants polymères, la proportion de ces agents favorisant la pénétration étant de préférence de 5 à 20% en poids.

11. Système thérapeutique transdermique sous forme d'emplâtre, présentant une couche arrière imperméable à la ou aux substances actives, un réservoir contenant une ou des substances actives à base d'une matrice polymère et une feuille de protection amovible, **caractérisé en ce que** le réservoir contenant une ou des substances actives est un auto-adhésif selon l'une quelconque des revendications 1 à 10.

12. Utilisation d'un auto-adhésif selon l'une ou plusieurs des revendications 1 à 7, pour la préparation de pansements rapides, d'emplâtres de fixation, de sparadraps, de pansements ou d'électrodes auto-adhésives.

13. Utilisation d'un auto-adhésif selon l'une ou plusieurs des revendications 1 à 10 pour la préparation d'emplâtres contenant une ou des substances actives pour l'administration transdermique de substances actives pharmaceutiques.

14. Utilisation selon la revendication 13, **caractérisée en ce que** les emplâtres contenant une ou des substances actives contiennent des substances actives pharmaceutiques acides ou basiques ou leurs sels pharmaceutiquement acceptables.
